# EUROPEAN PATENT APPLICATION

(11) **EP 1 915 944 A1**
(43) Date of publication of application: **30.04.2008**
(21) Application number: 07254237.6
(22) Date of filing: 25.10.2007
(51) Int. Cl.: A61B 5/145, A61B 5/00, G09B 5/00

(54) **Method for tutoring a user during use of a system for determining an analyte in a bodily fluid sample**

(30) Priority: 26.10.2006 US 553314
(71) Applicant: Lifescan Scotland Limited, Inverness-Shire IV2 3ED (GB)
(72) Inventor: Mcevoy, Mary, Belmont California 94002 (US); Cheney, Marcia, No. 29 Menlo Park California, 94025 (US); Newstadt, Tracy, San Francisco California 94116 (US); Newmark, Richard J., Inverness-shire IV24TA (GB)
(74) Representative: Tunstall, Christopher Stephen

(57) **Abstract**

A method for tutoring a user during use of a system for determining an analyte (for example, glucose) in a bodily fluid sample (e.g., a whole blood sample) include activating a system for determining an analyte in a bodily fluid sample. The system that is activated includes a meter and at least one analytical test strip configured for the application of the bodily fluid sample thereon and for insertion into the meter for subsequent determination of the analyte. Furthermore, the meter includes a graphics-based step-by-step tutorial module with a user interface, a microprocessor unit and a memory unit storing a step-by-step tutorial that includes graphics-based images depicting use of the system. In addition, the user interface, microprocessor unit and memory unit are operatively linked and configured for sequential display of the graphics-based images of the step-by-step tutorial to the user via, for example, a display screen. The method also includes tutoring the user in proper use of the system by sequentially displaying the graphics-based images of the step-by-step tutorial on the user interface.

## Description

### BACKGROUND OF THE INVENTION

**1. Field of the Invention**

The present invention relates, in general, to medical devices and, in particular, to systems and methods for determining an analyte in a bodily fluid sample.

**2. Description of the Related Art**

The determination (e.g., detection and/or concentration measurement) of an analyte in a bodily fluid sample is of particular interest in the medical field. For example, it can be desirable to determine glucose, cholesterol, acetaminophen and/or HbA1c concentrations in a sample of a bodily fluid such as urine, blood or interstitial fluid. Such determinations can be achieved using systems (also referred to herein as "kits") that employ analytical test strips based on, for example, photometric or electrochemical techniques, along with an associated meter. For example, the OneTouch^{®} Ultra^{®} whole blood testing kit, available from LifeScan, Inc., Milpitas, USA, employs an electrochemical-based analytical test strip for the determination of blood glucose concentration in a whole blood sample.

Such systems can be relatively complex with a plurality of buttons, an integrated analytical test strip dispenser, and multiple software-based capabilities. Therefore, users of such systems are typically provided with a written operating manual for the system. Depending on the complexity of the system, a user may need to devote significant time and concentration before they understand and have memorized the manual's information and are able to successfully operate the system.

### BRIEF DESCRIPTION OF THE DRAWINGS

A better understanding of the features and advantages of the present invention will be obtained by reference to the following detailed description that sets forth illustrative embodiments, in which the principles of the invention are utilized, and the accompanying drawings, of which:

FIG. 1 is a simplified block diagram of a system for determining an analyte in a bodily fluid sample according to an exemplary embodiment of the present invention;

FIG. 2 is a simplified front view of a meter and analytical test strip as can be included in a system according to exemplary embodiments of the present invention;

FIG. 3 is a simplified depiction of a sequence of main menu and step-by-step graphics and text-based tutorial images as can be stored and displayed by an exemplary embodiment of the present invention;

FIGs. 4A and 4B are additional graphics and text-based images that can be displayed on a graphical user interface employed in exemplary embodiments of the present invention; and

FIG. 5 is a flow diagram depicting stages in a process for tutoring a user during use of a system for determining an analyte in a bodily fluid sample according to an exemplary embodiment of the present invention.

### DETAILED DESCRIPTION OF EXAMPLARY EMBODIMENTS OF THE INVENTION

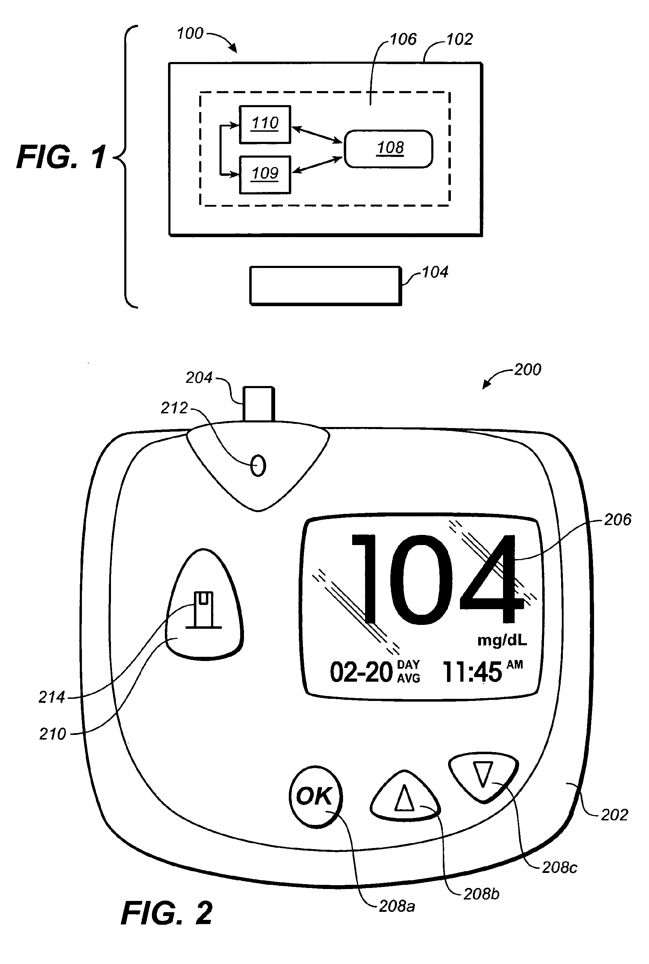

FIG. 1 is a simplified block diagram of a system 100 for determining an analyte (such as glucose) in a bodily fluid sample (e.g., a whole blood sample) according to an exemplary embodiment of the present invention. System 100 includes a meter 102 and at least one analytical test strip 104.

Meter 102 includes a graphics-based step-by-step tutorial module 106 (encompassed within the dashed line of FIG. 1) with a user interface 108, a microprocessor unit 109 and a memory unit 110. Memory unit 110 is configured for storing graphics-based and, optionally, text-based images of a step-by-step tutorial depicting use of system 100. Microprocessor unit 109 is configured to coordinate and control the various elements of graphics-based step-by-step tutorial module 106 including, at least, control and coordination between memory unit 110 and user interface 108.

User interface 108 is configured to display the graphics-based images and, optionally, text-based images of the step-by-step tutorial stored in memory unit 110. Such a step-by-step tutorial can be displayed, for example, as a sequence of static graphics-based and text-based images or as a sequential display of animated images containing a combination of animated graphics-based images and text. The sequence of graphics-based images depicts the proper use of the system for determining an analyte in a bodily fluid sample in a clear and intuitive way, thereby beneficially tutoring a user in use of the system. Since the images are displayed in a sequential manner, a user is always tutored in the proper and complete step-by-step use of the system. The sequential display thus serves, through repetition, to beneficially reinforce the proper and complete use of the system in a user's memory.

Once apprised of the present disclosure, one skilled in the art will recognize that graphics-based images employed in embodiments of the present invention are illustrative, pictorial, diagrammatic and/or simplified in nature and are not, therefore, necessarily accurate with respect to all mechanical or visual details and/or in scale. However, such graphics-based images are sufficiently accurate and detailed for the intended purpose, namely for the purpose of tutoring a user in the proper step-by-step operation of a system for determining an analyte in a bodily fluid sample.

Analytical test strip 104 is configured for the application of a bodily fluid sample thereon and for insertion in meter 102 for subsequent determination of an analyte in the bodily fluid sample. Analytical test strip 104 can be any suitable analytical test strip including, for example, an electrochemical-based or photometric-based analytical test strip. As noted below, meter 102 can employ any suitable technique known to those of skill in the art for determining the analyte in conjunction with analytical test strip 104. Conventional analytical test strips that could be employed in the present invention are described, for example, in U.S. Patent Nos. 5,708,247; 6,733,655; 5,753,452; 6,168,957; 6,555,061; 6,716,577; 6,723,500 and 6,241,862, each of which is hereby fully incorporated by reference.

User interface 108, microprocessor unit 109 and memory unit 110 are in operatively linked and configured for sequential display of the graphics-based step-by-step tutorial to a user during use of system 100. Such operative linking is depicted by doubleheaded arrows in FIG. 1.

Meter 102 can employ any suitable analytical technique or techniques to determine the analyte in the bodily fluid sample including, for example, techniques employed in current commercially available meters. Such techniques include, but are not limited to, photometric and electrochemical-based techniques. Once apprised of the present disclosure, one skilled in the art will recognize various manners by which conventional meters could be adapted to implement an embodiment of the present invention. For example, a microprocessor unit, memory unit and a user interface as described herein could be suitably integrated with an otherwise conventional meter to implement an embodiment of the present invention. Conventional meters that could be modified for use in the present invention are described in, for example, U.S. Patent Nos. 6,780,645; 5,605,837 and 6,576,416 and International Application PCT/US2005/047552 (published as WO2006/072035A1 on July 6, 2001), each of which is incorporated fully herein by reference.

User interface 108 of graphics-based step-by-step tutorial module 106 can be any suitable user interface and can include, for example, a display screen (not depicted in FIG. 1) and user operable buttons (also not depicted in FIG. 1). Such a display screen can be any suitable display screen known to those of skill in the art including a liquid crystal display (LCD) based display screen. Suitable display screens include, without limitation, display screens that are configured for displaying static graphics-based images (both with and/or without associated text) and animated graphics-based images (both with and/or without associated text).

Memory unit 110 of graphics-based step-by-step tutorial module 106 can be any suitable memory unit known to those of skill in the art including, for example, solid state nonvolatile memory (NVM) units. Moreover, the graphics-based and/or text-based images of the graphics-based step-by-step tutorial module can be stored within memory unit 110 using any suitable conventional format including, for example, software-based formats.

Microprocessor unit 109 of graphics-based step-by-step tutorial module 106 can be any suitable microprocessor unit known to those of skill in the art including, for example, configurable microprocessor units.

FIG. 2 is a simplified front view of a system 200 for determining an analyte (such as glucose) in a bodily fluid sample (e.g., a whole blood sample) according to an embodiment of the present invention. System 200 includes a meter 202 and at least one analytical test strip 204.

Meter 202 includes an integrated (i.e., embedded) graphics-based step-by-step tutorial module with a user interface. The user interface includes display 206 and user operable buttons 208a, 208b and 208c. The graphics-based step-by-step tutorial module also includes a microprocessor unit and a memory unit (both of which are not visible in the view of FIG. 2).

Meter 202 also includes a test strip actuation button 210 and an indicator light 212. Moreover, meter 202 includes an analytical test strip dispenser (not visible in the FIG. 2) containing a plurality of analytical test strips (for example, ten analytical test strips). The analytical test strip dispenser of meter 202 is configured such that a single analytical test strip is dispensed upon a user pressing test strip actuation button 210. In the embodiment of FIG. 2, test strip actuation button 210 is marked with an icon 214 depicting an analytical test strip protruding lengthwise the meter, thereby giving a preview of what the user will see after pressing test strip actuation button 210. Conventional analytical test strip dispensers that could be employed in embodiments of the present invention are described in, for example, U.S. Patent No. 6,908,008 and U.S. Patent Application Publication No. 2005/0118062A1 published on June 2, 2005, International Application Nos. PCT/GB2005/002534 (published as WO2006/000818A1 on January 5, 2006); PCT/GB2005/002518 (published as WO2006/000809A1 on January 5, 2006), PCT/GB2005/002500 (published as WO2006/000794A1 on January 5, 2006), PCT/GB2005/002497 (published as WO2006/000792A1 on January 5, 2006) and PCT/IB2005/004021 (published as WO2006/035322A2 on April 6, 2006), each of which is incorporated fully herein by reference.

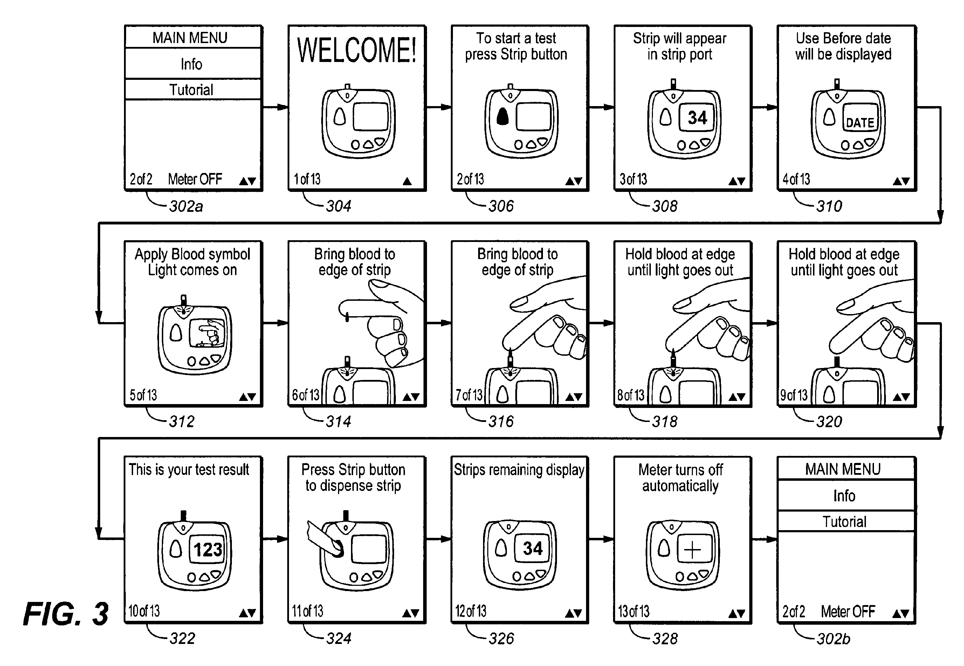

FIG. 3 is a simplified depiction of a sequence 300 of main menu (302a and 302b) and thirteen graphics-based and text-based images of a step-by-step tutorial (304 through 328) as can be stored and sequentially displayed by an exemplary embodiment of the present invention. The following description of FIG. 3 includes various beneficial non-limiting features of embodiments of the present invention.

Referring to FIGs. 1 and 3, when meter 202 is activated for the first time and for a predetermined number of times thereafter (for example, ten to thirty times thereafter) graphics and text-based images of the step-by-step tutorial are sequentially displayed on user interface 108. Alternatively, or following the predetermined number of meter activations, the graphics-based tutorial module can be activated by user selection from a main menu screen as depicted in image 302a of FIG. 3.

Images 304 through 328 of FIG. 3 are non-limiting examples of graphics-based and text based images that can be employed in exemplary embodiments of the present invention. The sequential images of FIG. 3 can be summarized as follows:
Image 302a - a main menu screen;
Image 304 - includes 'Welcome' text and a graphics-based image of meter 102;
Image 306 - includes text 'To start a test press the strip button', and a test strip actuation of the meter is highlighted in the image;
Image 308 - includes, in a graphics-based manner, a number in large font within the meter, an analytical test strip in the test position, and also includes the text 'Strip will appear in strip port';
Image 310 - includes the expiry date on the screen of the meter and associated text;
Image 312 - depicts, in a graphics-based manner, the meter with an indicator light thereon illuminated, a user's hand and a meter within the display window of the meter, while also depicting associated text;
Images 314, 316, 318 and 320 depict, in a sequential animated graphics-based manner, the application of a whole blood sample from a user's lanced index finger to an analytical test strip and associated text with the meter, hand, blood drop and indicator light, shown in an ideal position and/or orientation for correct sample application to the analytical test strip.

In particular, image 314 shows an exemplary embodiment of a graphics-based image of a hand with the middle, ring and small finger curled in towards the palm and only the index finger projecting outwards with a circular blood droplet on the end of the index finger. Below this image is a graphics-based outline of a portion of a meter with an analytical test strip protruding from a delivery port, ready to accept a blood sample. The indicator light of the meter is also shown illuminated. Image 314 can be sequentially displayed (i.e., sequentially displayed after the display of images 304 through 312), for example, to a user following actuation of an analytical test strip to the delivery port, and indicates to the user that the meter is in a state ready to receive a sample. In image 314, the hand and meter are separated.

In particular, image 316 is an image somewhat similar to image 314, however, in image 316 the index finger with blood droplet is contacting a sample application zone of the analytical test strip. In the embodiment of FIG. 3, the graphics-based image of the hand is animated and moves between the separated position shown in image 314 and the contacting position shown in image 316, while the meter is waiting to receive a sample. This sequence of animations can, if desired, continue in a repeated manner until a sample is applied to the analytical test strip and the meter recognizes correct and sufficient sample application, following which the remaining images are sequentially displayed. Such animation is provided as an intuitive and direct way to tutor (i.e., educate) the user, and to invite the user to imitate the actions shown in the images.
Image 322 depicts a meter with an example test result displayed thereon;
Image 324 - depicts the manner in which a user can depress the test strip actuation button (shown highlighted) to eject the used analytical test strip;
Image 326 - includes a depiction of the remaining number of analytical test strips;
Image 328 depicts the meter turning off automatically and includes a graphics-based image of a meter with a blank display. If desired, the image can also employ text to instruct a user to consult the system's operating manual prior to system use.
Image 302b - depicts the main menu for system 100.

Images 304 through 320 of FIG. 3 depict, in an intuitive step-by-step (and sequential) manner, essentially the complete use of system 100 for determining an analyte in a bodily fluid sample. The steps depicted range from obtaining an analytical test strip to disposing of the used analytical test strip. Meter 102 can be configured such that a user has the option of moving sequentially forwards or sequentially backwards (i.e., scrolling) through the images by operation of a user operable button.

Although the graphics and text-based images of FIG. 3 pertain to the determination of blood glucose concentration in a whole blood sample, once apprised of the present disclosure it would be obvious to one skilled in the art that a systems according to exemplary embodiments of the present invention encompass the determination of another analytes (e.g., HbA1c, lactate and cholesterol) and samples of other bodily fluids (e.g., interstitial fluid (ISF) or urine).

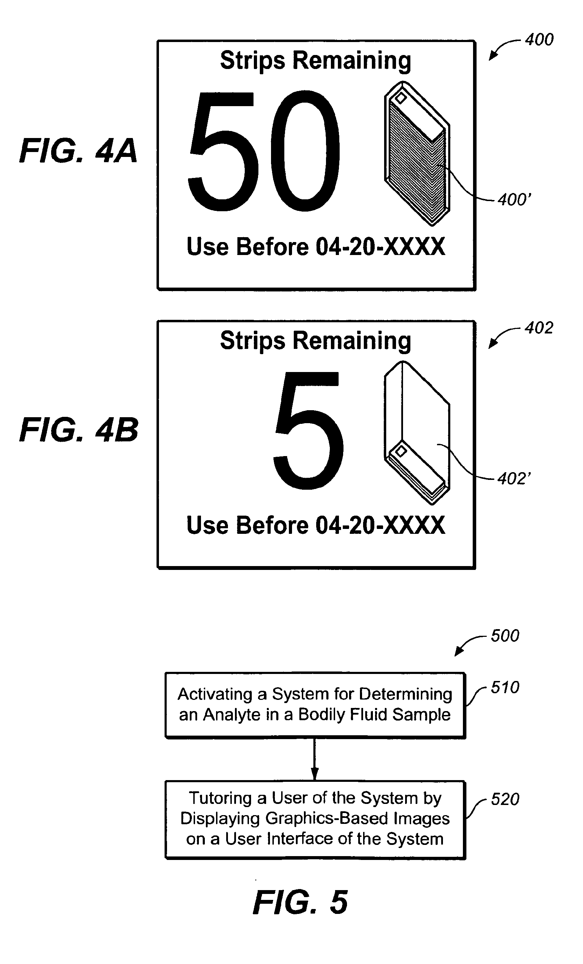

If desired, user interface 108 of system 100 can also be employed to display the number of analytical test strips remaining within system 100. In this regard, FIGs. 4A and 4B are additional graphics and text-based images that can be displayed on a graphical user interface employed in exemplary embodiments of the present invention.

FIG. 4A is an image 400 depicting fifty analytical test strips remaining and FIG. 4B is an image 402 depicting 5 analytical test strips remaining. Each of FIGs. 4A and 4B depict the number of analytical test strips remaining in text of a relatively large font size, and also include a graphics-based image (i.e., 400' and 402') depicting an analytical test strip stack height in a relative manner. In the embodiment of FIGs 4A and 4B, the graphics-based image is of a pseudo three dimensional rectangular shaped stack representing a cartridge, with a black portion indicating the height filled with analytical test strips. The images of FIGs. 4A and 4B also include text related to the expiration date of analytical test strips contained with the meter with such expiration date having, for example, been previously supplied to the meter by a user or via electronic means.

Images such as those of FIGs. 4A and 4B can be optionally displayed to the user whenever the meter is activated and/or before a meter is powered off. Providing a reminder of the number of analytical test strips is intended to aid a user in remembering to have a sufficient supply of analytical test strips.

FIG. 5 is a flow diagram depicting stages in a method 500 for tutoring a user during use of a system for determining an analyte in a bodily fluid sample according to an exemplary embodiment of the present invention. Method 500 includes activating a system for determining an analyte in a bodily fluid sample (see step 510 of FIG. 5).

The system that is activated includes a meter and at least one analytical test strip configured for the application of the bodily fluid sample thereon and for insertion into the meter for subsequent determination of the analyte. Furthermore, the meter includes a graphics-based step-by-step tutorial module with a user interface, a microprocessor unit and a memory unit storing a step-by-step tutorial that includes graphics-based images depicting use of the system. In addition, the user interface, microprocessor unit and memory unit are operatively linked and configured for sequential display of the graphics-based images of the step-by-step tutorial to the user via, for example, a display screen.

At step 520 of method 500, the user is tutored by displaying the graphics-based images of the step-by-step tutorial on the user interface in a sequential manner.

Once apprised of the present disclosure, one skilled in the art will recognize that methods according to embodiments of the present invention can include steps that carry out functional characteristics of embodiments of systems according to the present invention. For example, the tutoring step can occur automatically upon activation of the meter in the manner described herein and/or the tutoring step can include a user scrolling through graphics-based images. Moreover, the activating step of methods according to the present invention can include the activation of any suitable system described with respect to system embodiments of the present invention.

It should be understood that various alternatives to the embodiments of the invention described herein may be employed in practicing the invention. It is intended that the following claims define the scope of the invention and that structures and methods within the scope of these claims and their equivalents be covered thereby.

## Claims

1. A method for tutoring a user during use of a system for determining an analyte in a bodily fluid sample, the method comprising:
activating a system for determining an analyte in a bodily fluid sample, the system including:
a meter that includes:
a graphics-based step-by-step tutorial module with:
a user interface;
a memory unit storing a step-by-step tutorial that includes graphics-based images depicting use of the system, and
a microprocessor unit configured for coordination and control of at least the user interface and the memory unit; and
at least one analytical test strip configured for:
the application of a bodily fluid sample thereon; and
insertion in the meter for subsequent determination of an analyte in the bodily fluid sample;
and wherein the user interface, microprocessor unit and memory unit are in operatively linked and configured for sequential display of the graphics-based images of the step-by-step tutorial to a user; and
tutoring the user in use of the system by sequentially displaying the graphics-based images of the step-by-step tutorial on the user interface.

2. The method of claim 1 wherein the tutoring step further includes sequentially displaying text stored in the memory unit.

3. The method of claim 1 wherein the tutoring step includes sequentially displaying animated graphics-based images of the step-by-step tutorial stored in the memory unit.

4. The method of claim 1 wherein the tutoring step occurs automatically upon a first activation of the meter and upon a predetermined number of subsequent activations of the meter.

5. The method of claim 4 wherein the predetermined number of subsequent activations of the meter is in the range of one subsequent activation to thirty subsequent activations.

6. The method of claim 5 wherein the meter is configured such that the user interface sequentially displays the graphics-based images upon user request via the user interface following the predetermined number of subsequent activations of the meter.

7. The method of claim 1 wherein the tutoring step tutors a user by displaying graphics-based images that depicting use of the meter and analytical test strip for the determination of glucose in a whole blood sample.

8. The method of claim 7 the tutoring step includes a user scrolling through the graphics-based images in at least one of scrolling sequentially forward and scrolling sequentially backward.

9. The method of claim 1 wherein the system is configured for determining glucose in a whole blood sample.

10. The method of claim 1 wherein the meter further includes at least one user operable button for controlling the sequential display of the graphics-based images on the display screen and wherein the tutoring step includes user control of the sequential display via the at least one user operable button.
